# EUROPEAN PATENT SPECIFICATION

(11) **EP 4 104 782 B1**
(45) Date of publication and mention of the grant of the patent: **20.08.2025**
(21) Application number: 21753554.1
(22) Date of filing: 27.01.2021
(51) Int. Cl.: A61B 18/14

(54) **ELECTROSURGICAL ELECTRODE**
ELEKTROCHIRURGISCHE ELEKTRODE
ÉLECTRODE ÉLECTROCHIRURGICALE

(30) Priority: 13.02.2020 JP 2020022896
(43) Date of publication of application: 21.12.2022
(73) Proprietor: Nihon Parkerizing Co., Ltd., Tokyo 103-0027 (JP)
(72) Inventor: UCHIDA, Junichi, Tokyo 103-0027 (JP); YOROZU, Takayuki, Tokyo 103-0027 (JP); KATSURAYA, Ryoko, Tokyo 103-0027 (JP); UMEHARA, Noritsugu, Nagoya-shi, Aichi 464-0812 (JP); TOKOROYAMA, Takayuki, Nagoya-shi, Aichi 458-0015 (JP); MURASHIMA, Motoyuki, Nagoya-shi, Aichi 464-0823 (JP)
(74) Representative: Jones, Nicholas Andrew
(86) International application number: PCT/JP2021/002802
(87) International publication number: WO 2021/161785

(56) References cited:
- WO-A1-2020/027341
- WO-A1-2020/027341
- JP-A- 2000 333 968
- US-A- 5 380 320
- US-A- 5 702 387
- US-A- 6 070 444
- US-A1- 2019 185 370

## Description

### [Technical Field]

The present invention relates to an electrosurgical electrode for an electrosurgical instrument that is used as a medical device to be used for surgery performed on a living tissue.

### [Background Art]

In a surgical procedure, an electrosurgical instrument (so-called electric cautery) that disperses, from an electrosurgical electrode to a living tissue, a high-frequency current generated from a main body thereof to be able to perform hemostasis (coagulation) or incision is indispensable. As one of problems caused by using the electric cautery, a problem of "burn eschar" in which a carbide of a living tissue or the like adheres to a leading end of the electric cautery is known. To address the problem, there has been proposed a method of mass-producing a plurality of electrodes each connectable to an appropriate power source for a surgical procedure, and the method includes the steps of: preparing an electrically conductive stock material having a shape and dimensions which is capable of forming a plurality of electrode blanks; coating at least a part of the stock material described above with a non-adhesive layer; and forming the plurality of coated electrode blanks (see Patent Literature 1). US6070444 discloses a method of mass manufacturing a plurality of electrodes coated with a non-stick layer for reducing eschar buildup and the like on the working tip of an electrode during electrosurgical procedures and facilitating ease of cleaning the tip. US5380320 discloses an electrosurgical instrument having a conductive elongated member, a connector for connecting an external electrical power supply to the proximal end of the elongated member, an electrosurgical implement at the distal end of the elongated member, and an insulative parylene coating covering the elongated member. US5702387 discloses a design and method of manufacture is disclosed for an electrosurgical electrode with a silicone coating. The coating resists the buildup of eschar.

### [Citation List]

### [Patent Literature]

[PTL 1] Japanese Patent Application Publication No. 2000-333968

### [Summary of Invention]

### [Technical Problem]

However, since the electrosurgical electrode described in Patent Literature 1 has the non-adhesive layer, heat is unlikely to be dissipated from a surface of the electrosurgical electrode, and a temperature of a leading end portion of the electrosurgical electrode, i.e., an operating region that comes closest to a living tissue to perform incision and/or hemostasis on the living tissue may excessively rise. When the temperature of the leading end portion of the electrosurgical electrode has excessively risen, there is a problem that heat more than necessary is transferred from the operating region to an affected area and excessive heat invasion occurs, which damages a healthy tissue.

The present invention is intended to solve such a problem, and addresses the problem of providing an electrosurgical electrode that can suppress an excessive temperature rise in an operating region.

### [Solution to Problem]

The present inventors have studied to solve the problem described above, and have found that the problem can be solved by an electrosurgical electrode according to claim 1.

### [Advantageous Effects of Invention]

The present invention can provide an electrosurgical electrode capable of suppressing an excessive temperature rise in a portion that can be an operating region.

### [Brief Description of Drawings]

[Fig. 1]

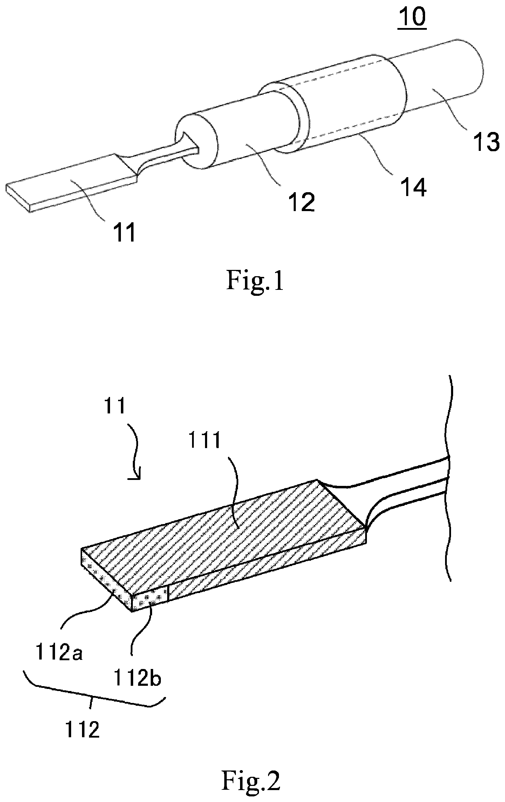

   Fig. 1 is a schematic diagram illustrating an example (blade electrode) of an electrosurgical electrode (electric cautery).
[Fig. 2]
   Fig. 2 is a schematic diagram illustrating an example of a coating portion (A) and a region (B) of the electrosurgical electrode.

### [Description of Embodiments]

An embodiment is an electrosurgical electrode to be used for surgery performed on a living tissue, and the electrosurgical electrode comprises a principal body capable of emitting a high-frequency energy. A surface of the principal body includes a coating portion (A) coated with a resin coating having a thickness of not less than 10 µm and a region (B) uncoated or coated with an oxide coating and/or a resin coating having a thickness of not more than 1.0 µm. The region (B) is an effective surface capable of dispersing the high-frequency energy from the principal body to the living tissue.

### <Electrosurgical Electrode>

The electrosurgical electrode is an electrode to be removably attached to a leading end of an electrosurgical instrument such as a so-called electric cautery. By emitting a high- frequency wave from a principal body of the electrode to a living tissue, it is possible to perform hemostasis (coagulation) or incision on the living tissue. The electrosurgical electrode is formed of an electrically conductive material. More specifically, an iron-based metal material, a zinc-plated metal material, an aluminum-based metal material, a magnesium-based metal material, a nickel-based metal material, a titanium-based metal material, a zirconium-based metal material, a copper-based metal material, a tin-based metal material, a tungsten-based metal material, a chromium-based metal material, a manganese- based metal material, a molybdenum-based metal material, a cobalt-based metal material, or the like can be used but, more preferably, stainless steel is used. Typical representatives of the electrosurgical instrument to which the electrosurgical electrode is to be attached include an electric cautery such as a monopolar cautery or a bipolar cautery, a laparoscope, and the like. As the monopolar cautery, a cautery having a shape of a blade type, an annular structure type, a rod-shaped structure type having a needle structure in a leading end thereof, a spherical structure type, or the like can be listed. Fig. 1 illustrates a schematic diagram of an example of the electrosurgical electrode.

Fig. 1 is a schematic diagram illustrating an example of a blade-type electrosurgical electrode having a plate-shaped leading end portion.

The electrosurgical electrode 10 is a member detachable from a main body of an electrosurgical instrument not shown. The electrosurgical electrode 10 comprises an electrical connection portion 13 to be electrically connected to the main body of the electrosurgical instrument, a principal body 11 to be brought closer to a living tissue to emit a high-frequency wave, and an intermediate portion 12 connecting the electrical connection portion 13 and the principal body 11.

Fig. 2 is an enlarged view illustrating the principal body 11 in Fig. 1 in enlarged relation. In the present embodiment, a surface of the principal body 11 includes a coating portion (A) 111 (hatched region in the figure) coated with a resin coating having a thickness of not less than 10 µm and a region (B) 112 (dotted region in the figure) uncoated or coated with an oxide coating and/or a resin coating having a thickness of not more than 1.0 µm.

The principal body 11 has a substantially rectangular shape having a front surface serving as a main surface, a back surface opposing the front surface, and a leading end portion (leading end surface) and a side surface portion (both side surfaces) each lying interposed between the front surface and the back surface. The front end portion and a part of the side surface portion include the region (B) uncoated or coated with the oxide coating and/or the resin coating having the thickness of not more than 1.0 gm. The region (B) is preferably present in the leading end portion and in the side surface portion, but not limited only to the leading end portion and the side surface portion, and the region (B) may also be present in a part of the front surface and/or the back surface. Specifically, a leading end portion 112a and a region 112b of the side surface portion continued to the leading end portion may serve as the region (B). Note that, in the present embodiment, the region 112b is assumed to be the part of the side surface portion, but may also be the entire side surface portion.

Alternatively, the leading end portion 112a may serve as an operating region that comes closest to a living tissue to perform incision or hemostasis on the living tissue. Note that the principal body of the blade-type electrosurgical electrode illustrated in Fig. 2 has the rectangular shape, but not limited thereto. The blade-type electrosurgical electrode may have any shape having the front surface, the back surface, the leading end portion, and the side surface portion. In addition, sizes (areas) of the front surface and the back surface may be the same or different from each other. In the present description, the main surface refers to a plane having a largest area.

In the present embodiment, a ratio (β1/αI) of an area (βl) of the region (B) 112 described above to an area (αI) of the coating portion (A) 111 described above is in a range of 0.01 to 0.5.

By setting the value of (βl/αI) to be in the range described above at the surface of the principal body 11, the electrosurgical electrode capable of suppressing an excessive temperature rise in the portion that may serve as the operating region is provided. The range of (βl/αI) is preferably not less than 0.027 or preferably not more than 0.476.

The coating portion (A) 111 described above may also be coated with a resin coating having a thickness of not less than 10 µm. For the resin coating, a fluorine-containing resin such as Teflon (registered trademark) or PTFE, a silicone resin, or the like can be used, and the resin coating preferably has an anti-adhesive function (antifouling function). The resin coating may comprise a single layer or a lamination layer of multiple layers. When the resin coating includes the lamination layer of multiple layers, the multiple layers may be made of the same resin or different resins. Alternatively, it may also be possible to have an oxide coating between the resin coating and the principal body.

The resin coating can be formed by bringing a resin composition comprising a fluorine-containing resin or a silicone resin into contact with the principal body 11. The resin composition may also comprise an additive such as a filler, a plasticizer, a mold-release agent, or a cross-linking agent. The thickness of the resin coating may be not less than 20
µm and not less than 30 µm. Note that an upper limit of the thickness of the resin coating is not particularly limited, and the thickness of the resin coating may also be not more than 1 mm.

The region (B) described above is a region uncoated or coated with the oxide coating and/or the resin coating having the thickness of not more than 1.0 µm. The region (B) is an effective surface capable of dispersing the high-frequency energy from the principal body described above to a living tissue. Accordingly, the region (B) includes the operating region that comes closest to the living tissue to perform incision and/or hemostasis on the living tissue during use of the electrosurgical instrument to which the electrosurgical electrode is attached.

The oxide coating and/or the resin coating that may be included in the region (B) is extremely thin compared to the resin coating (A), and a thickness thereof is not more than 1.0 µm, but may also be not more than 0.9 µm, not more than 0.8 µm, not more than 0.7 µm, not more than 0.6 µm, not more than 0.5 µm, not more than 0.4 µm, not more than 0.3 µm, not more than 0.2 µm, not more than 0.1 µm, or not more than 0.05 µm.

In another embodiment, the region (B) described above may include a surface in contact with the entire or a part of operating region of the principal body described above that comes closest to a living tissue to perform incision and/or hemostasis on the living tissue, and the surface may include a region bounded by a line in contact with the operating region and by a line resulting from a vertical translational shift of at least 2.0 mm from the line along the surface. The presence of the region (B) in such a range can prevent an excessive rise of a temperature due to heat built up in the vicinity of the operating region that performs incision and/or hemostasis on the living tissue. Preferably, the region (B) may include a region bounded by a line resulting from a vertical translational shift of at least 3.0 mm from the line along the surface.

As described previously, the region (B) described above may also include the leading end portion 112a, include the part 112b of the side surface portion, or include the part of the front surface and/or the back surface. Preferably, the region (B) described above includes the leading end portion 112a, further includes the part 112b of the side surface portion, and further includes the part of the front surface and/or the back surface. In other words, the priority order of the portions that may serve as the region (B) described above are the leading end portion 112a, the part 112b of the side surface portion, and the front and back surfaces, in that order. In addition, on each of the side surface portion, the front surface, and the back surface, a part of the region (B) is preferably present so as to extend from the leading end portion.

The region bounded by the line in contact with the operating region and by the line resulting from the vertical translational shift of at least 2.0 mm from the line along the surface may be or may not be coated with the oxide coating and/or the resin coating having the thickness of not more than 1.0 µm.

The principal body of the electrosurgical electrode can be produced by, e.g., the following method. By way of example, a degreasing treatment, a surface preparation treatment, and the like are performed as necessary on the principal body, and then the principal body is immersed in a resin coating composition to apply a resin coating to the entire surface of the principal body. Then, of the principal body, the leading end portion, the side surface portion, and intended parts of the front and back surfaces are scraped off as necessary to thereby, and the principal body can be produced. The degreasing treatment, the surface preparation treatment, and the like are not particularly limited as long as the oxide coating and/or the resin coating can be formed thereby, and known methods can be used. In addition, the resin coating composition is not particularly limited as long as the resin coating composition is a resin composition comprising a fluorine-based resin or a silicone-based resin.

### <Shape of Principal Body>

A shape of the principal body 11 is not particularly limited, and other than the blade shape of the principal body 11 illustrated in Fig. 2, it can be annular-type (loop-type) shape, a ball-type shape, or a needle-type shape. These are electrosurgical electrodes to be used as cautery-tip electrodes of electric cauteries. Besides, an electrosurgical electrode to be attached to a laparoscope, such as a wire L-shaped hook type, a straight spatula type, a wire J- shaped hook type, or a syringe type, may also be used. The principal body shown heretofore by way of example is a monopolar-type electrosurgical electrode, but may also be a bipolar-type electrosurgical electrode.

### <Electrical Connection Portion>

The electrical connection portion 13 is a portion of the electrosurgical electrode 10 to be electrically connected to the main body of the electrosurgical instrument. The electrical connection portion 13 is detachable from the main body of the electrosurgical
instrument and, typically, the electrical connection portion 13 and the main body of the electrosurgical instrument are configured to be interfittable with each other using an interfitting structure or the like. Note that the electrical connection portion is also formed of an electrically conductive material, and a forming material may be the same as or different from that of the principal body 11.

### <Intermediate Portion>

The intermediate portion 12 is a member connecting the principal body 11 and the electrical connection portion 13. To conduct electricity to the principal body 11, the intermediate portion 12 is required to be formed of an electrically conductive material, but a shape, a length, and the like of the intermediate portion 12 are not particularly limited.

The intermediate portion 12 may also have a coating 14. The coating 14 is a hardened material of a composition comprising a resin having an insulating property. As long as the coating 14 is in contact with the intermediate portion 12, a size, a thickness, a shape, and the like of the coating 14 are not particularly limited, either.

### Examples

The following will specifically show a function/effect of the present invention by using Examples. However, statements related to Examples described below are not intended to limit the scope of the present invention as defined in claim 1 in any way.

### <Production of Principal Body>

The blade-type electrosurgical electrode having the plate-shaped principal body 11 illustrated in Fig. 1 was prepared. A material of the prepared electrosurgical electrode and a size of a blade portion were as follows.
Material of Electrosurgical Electrode: Stainless Steel SUS304
Size of Blade Portion: 0.3 mm in Plate thickness, 17.0 mm in Length, 2.5 mm in Width, and 0.00009595 m² in Area

The blade portion of the electrosurgical electrode was immersed in ethanol (1st grade, manufactured by Junsei, Chemical Co., Ltd.), and an ultrasonic wave was applied thereto for 10 minutes to remove oil and contamination from over a surface thereof. Then, by drying the blade portion at 100 °C for 10 minutes, the adhering ethanol was removed.

To the blade portion from which the oil and contamination had been removed, a silicone composition (Product Name; ES-1002T manufactured by Shinetsu Chemical Industry Co., Ltd.) was applied using the following dispenser. Then, by drying the blade portion at a drying temperature of 150 °C for 30 minutes, the electrosurgical electrode having a silicone coating having a coating thickness of 70 µm was obtained. Dispenser (Desktop Robot): Product Name; ML-808GX, SM4000MEGAX-3A-SS manufactured by Musashi Engineering Co., Ltd.)

### <Evaluation Test>

Of the silicone coating with which the principal body 11 was coated, the coating on the leading end portion (112a in Fig. 2) was scraped off using a cutter knife. The principal body 11 in this state was assumed to be Comparative Example 1. Then, the coating on the part (112b in Fig. 2) of the side surface portion was scraped off using a cutter knife such that a distance from the leading end portion 112a has values shown in Table 1, and the principal bodies 11 in these states were assumed to be Examples 1 to 6. Note that the scraping off of the coating from over the side surface portion was performed on the both surfaces opposing each other.

Then, the coating on each of the front surface and the back surface of the principal body 11 produced in Example 6 was scraped off using a cutter knife such that the distance from the leading end portion has values shown in Table 1, and the principal bodies 11 in these states were assumed to be Examples 7 to 10 and Comparative Examples 2 and 3. In addition, the electrosurgical electrode having the principal body 11 from which the coating had not been scraped off at all was assumed to be Comparative Example 4.

For the electrosurgical electrodes in Examples 1 to 10 and Comparative Examples 1 to 4, maximum temperatures and temperature rises were measured. Specifically, each of the principal bodies 11(17 mm from the leading end portion) was inserted into a vessel containing pig's blood, and a temperature of the leading end portion of the principal body when the electrode was discharged with a power source output of 75 W for 5 seconds was measured using an infrared camera. The maximum temperature of the principal body was assumed to be a maximum temperature during 5-second measurement of the principal body 11. In addition, as a temperature rise per second, a temperature rising speed per unit time was calculated from a measured temperature change. Evaluation criteria were set as follows. A result is shown in Table 1.
<Maximum Temperature of Principal Body during 5-Second Discharge>
   Excellent: Not more than 270 °C
   Good: More than 270 °C and not more than 320 °C
   Acceptable: More than 320 °C and not more than 350 °C
   Unacceptable: More than 350 °C
<Temperature Rise per Second>
   Excellent: Not more than 90 °C/s
   Good: More than 90 °C/s and not more than 100 °C/s
   Acceptable: More than 100 °C/s and not more than 120 °C/s
   Unacceptable: More than 120 °C/s

**[Table 1]**

| | Distance from leading end portion of effective surface (B) at side surface (mm) | Distance from leading end portion of effective surface (B) at front surface and back surfaces (mm) | β (m²) | α (m²) | β/α | Maximum Temperature (°C) | | Temperature Rise (°C/s) | |
|---|---|---|---|---|---|---|---|---|---|
| Example 1 | 2 | 0 | 0.00000195 | 0.00009595 | 0.0207447 | Excellent | 270 | Good | 93 |
| Example 2 | 3 | 0 | 0.00000255 | 0.00009595 | 0.0273019 | Excellent | 251 | Excellent | 86 |
| Example 3 | 4 | 0 | 0.00000315 | 0.00009595 | 0.033944 | Excellent | 248 | Excellent | 83 |
| Example 4 | 5 | 0 | 0.00000375 | 0.00009595 | 0.0406725 | Excellent | 239 | Excellent | 80 |
| Example 5 | 10 | 0 | 0.00000675 | 0.00009595 | 0.0756726 | Excellent | 250 | Excellent | 82 |
| Example 6 | 17 | 0 | 0.00001095 | 0.00009595 | 0.1288235 | Excellent | 250 | Excellent | 83 |
| Example 7 | 17 | 1 | 0.00001595 | 0.00009595 | 0.199375 | Excellent | 261 | Excellent | 87 |
| Example 8 | 17 | 2 | 0.00002095 | 0.00009595 | 0.2793333 | Excellent | 253 | Excellent | 85 |
| Example 9 | 17 | 3 | 0.00002595 | 0.00009595 | 0.3707143 | Excellent | 245 | Excellent | 82 |
| Example 10 | 17 | 4 | 0.00003095 | 0.00009595 | 0.4761538 | Excellent | 241 | Excellent | 81 |
| Comparative Example 1 | 0 | 0 | 0.00000075 | 0.00009595 | 0.0078782 | Unacceptable | 325 | Unacceptable | 108 |
| Comparative Example 2 | 17 | 6 | 0.00004095 | 0.00009595 | 0.7445455 | Unacceptable | 349 | Unacceptable | 118 |
| Comparative Example 3 | 17 | 17 | 0.00009595 | 0.00009595 | - | Unacceptable | 368 | Unacceptable | 123 |
| Comparative Example 4 | 0 | 0 | 0 | 0.00009595 | - | Unacceptable | 412 | Unacceptable | 132 |

While the present invention is described in detail with reference to specific embodiments, it is obvious to a person skilled in the art that various changes and modifications can be made without departing from the scope of the present invention as defined in the claims.

### [Reference Signs List]

- 10: Electrosurgical electrode
- 11: Principal body
- 111: Coating portion
- 112: Region
- 112a: Leading end portion
- 112b: Part of side surface portion
- 12: Intermediate portion
- 13: Electrical connection portion
- 14: Coating

## Claims

1. An electrosurgical electrode (10) to be used for surgery performed on a living tissue, the electrosurgical electrode (10) comprising:
a principal body (11) capable of emitting a high-frequency energy,
a surface of the principal body including a coating portion A (111) coated with a resin coating having a thickness of not less than 10 µm and a region B (112) uncoated or coated with an oxide coating and/or a resin coating having a thickness of not more than 1.0 µm, **characterised in that**:
(i) the region B (112) is an effective surface capable of dispersing the high-frequency energy from the principal body (11) to the living tissue, and
a ratio βl/αl of an area βl of the region B (112) to an area αl of the coating portion A (111) is in a range of 0.01 to 0.5; or,
(ii) one end of the principal body (11) is an end connecting to an electrical connection portion (13), and the other end of the principal body (11) is a leading end portion (112a) which is closest to the living tissue to perform incision and/or hemostasis on the living tissue, and
the region B (112) is an effective surface capable of dispersing the high-frequency energy from the principal body (11) to the living tissue, and includes the leading end portion (112a) of the principal body (11) and a region (112b) bounded by a first line in contact with the operating region and by a second line resulting from a translational shift of at least 2.0 mm from the first line towards the connecting portion (13) of the principal body (11).

## Patentansprüche

1. Elektrochirurgische Elektrode (10) zur Verwendung für Chirurgie, die an einem lebenden Gewebe durchgeführt wird, wobei die elektrochirurgische Elektrode (10) Folgendes umfasst:
einen Hauptkörper (11), der fähig ist, Hochfrequenzenergie abzugeben,
eine Oberfläche des Hauptkörpers, die einen Beschichtungsabschnitt A (111) einschließt, der mit einer Harzbeschichtung beschichtet ist, die eine Dicke von nicht weniger als 10 µm aufweist, und einen Bereich B (112), der unbeschichtet oder mit einer Oxidbeschichtung und/oder einer Harzbeschichtung beschichtet ist, die eine Dicke von nicht mehr als 1,0 µm aufweist, **dadurch gekennzeichnet, dass**:
(i) der Bereich B (112) eine effektive Oberfläche ist, die fähig ist, die Hochfrequenzenergie vom Hauptkörper (11) an das lebende Gewebe zu verteilen, und
ein Verhältnis β1/α1 einer Fläche β1 des Bereichs B (112) zu einer Fläche α1 des Beschichtungsabschnitts A (111) im Bereich von 0,01 bis 0,5 liegt; oder,
(ii) ein Ende des Hauptkörpers (11) ein Ende ist, das an einem elektrischen Anschlussabschnitt (13) angeschlossen ist, und das andere Ende des Hauptkörpers (11) ein vordere Endabschnitt (112a) ist, der am nächsten zum lebenden Gewebe liegt, um Inzision und/oder Hämostase an dem lebenden Gewebe durchzuführen, und
der Bereich B (112) eine effektive Oberfläche ist, die fähig ist, die Hochfrequenzenergie vom Hauptkörper (11) an das lebende Gewebe zu verteilen und den vorderen Endabschnitt (112a) des Hauptkörpers (11) und einen durch eine erste Linie in Kontakt mit dem operativen Bereich und durch eine zweite Linie, die aus einer Parallelverschiebung von mindestens 2,0 mm von der ersten Linie zum Anschlussabschnitt (13) des Hauptkörpers (11) hin resultiert, abgegrenzten Bereich (112b) einschließt.

## Revendications

1. Électrode électrochirurgicale (10) pour être réalisée pour une chirurgie réalisée sur un tissu vivant, l'électrode électrochirurgicale (10) comprenant :
un corps principal (11) capable d'émettre une énergie à haute fréquence,
une surface du corps principal incluant une partie de revêtement A (111) recouverte d'un revêtement en résine ayant une épaisseur non inférieure à 10 µm et une région B (112) non revêtue ou revêtue d'un revêtement d'oxyde et/ou d'un revêtement de résine ayant une épaisseur non supérieure à 1,0 µm, **caractérisée en ce que** :
(i) la région B (112) est une surface effective capable de disperser l'énergie haute fréquence du corps principal (11) vers le tissu vivant, et
un rapport β1/α1 d'une surface β1 de la région B (112) sur une surface α1 de la portion de revêtement A (111) est dans une plage de 0,01 à 0,5 ; ou,
(ii) une extrémité du corps principal (11) est une extrémité se connectant à une portion de connexion (13) électrique, et l'autre extrémité du corps principal (11) est une portion d'extrémité avant (112a) qui est la plus proche du tissu vivant pour effectuer une incision et une hémostase sur le tissu vivant, et
la région B (112) est une surface effective capable de disperser l'énergie à haute fréquence provenant du corps principal (11) vers le tissu vivant, et inclut la partie d'extrémité avant (112a) du corps principal (11) et une région (112b) délimitée par une première ligne en contact avec la région opératoire et par une deuxième ligne résultant d'un décalage translationnel d'au moins 2,0 mm par rapport à la première ligne vers la partie de connexion (13) du corps principal (11).
